# EUROPEAN PATENT APPLICATION

(11) **EP 2 039 376 A1**
(43) Date of publication of application: **25.03.2009**
(21) Application number: 08162010.6
(22) Date of filing: 07.08.2008
(51) Int. Cl.: A61L 15/20, A61L 15/44, A61F 13/15

(54) **Absorbent articles comprising an aglycone derivable from an iridoid glycoside**

(30) Priority: 13.09.2007 EP 07116288
(71) Applicant: The Procter & Gamble Company, Cincinnati OH 45202 (US)
(72) Inventor: Carlucci, Giovanni, 66100, Chieti (IT); Guarracino, Mario, 64028, Silvi (Teramo) (IT); Steffan, Silvia, 10042, Nichelino (Torino) (IT)
(74) Representative: Briatore, Andrea

(57) **Abstract**

An absorbent article comprising aglycone derivable from an iridoid glycoside and/or one or more derivative thereof have an improved body fluid retention properties.

## Description

### FIELD OF THE INVENTION

The present invention relates to absorbent articles which include an aglycone derivable from an iridoid glycoside and/or a derivative thereof to provide an enhanced effect in fluid retention.

### BACKGROUND OF THE INVENTION

Absorbent articles of personal hygiene are known in the art. Typical examples include sanitary napkins, panty liners, adult incontinence articles, infant diapers, paper towels, bath tissue and facial tissue. Such articles are often used to absorb and retain bodily fluids and other exudates excreted by the human body.

Fluids are often retained in absorbent articles within an absorbent element comprising absorbent materials which often include superabsorbent materials, such as absorbent gelling materials (AGM), usually in finely dispersed form, e.g. typically in particulate form. Conventional superabsorbent materials known in the art for use in absorbent articles typically comprise water insoluble, water swellable, hydrogel forming crosslinked absorbent polymers which are capable of absorbing large quantities of liquids and of retaining such absorbed liquids under moderate pressure. In general, absorbent articles comprising conventional absorbent gelling materials commonly have good absorption and retention characteristics to water and urine; however, there still remains room for improvement for absorption and retention towards certain liquids. In particular, proteinaceous or serous body fluids such as typically menses, blood, plasma, vaginal secretions, mucus or milk, require more time to be effectively absorbed and consequently, especially in case of large amount of fluids, these might not be retained by the article and may leak outside.

Therefore in some cases it may be desirable to provide absorbent articles which are able to prevent leakage of body fluids, especially proteinaceous body fluids, even in the case when large amounts of fluids are discharged.

### SUMMARY OF THE INVENTION

The present invention provides an absorbent article comprising an aglycone derivable from an iridoid glycoside and/or a derivative thereof. Absorbent articles according to the present invention have an improved fluid retention properties.

### DETAILED DESCRIPTION OF THE INVENTION

The term "absorbent article" is used herein in a very broad sense including any article able to receive and/or absorb and/or contain and/or retain fluids and/or exudates, especially bodily fluids/bodily exudates. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). Typical disposable absorbent articles according to the present invention are diapers, surgical and wound dressings and perspiration pads, incontinence pads, as well as absorbent articles for feminine hygiene like sanitary napkins, panty liners, tampons, interlabial devices or the like. Absorbent articles suitable for use in the present invention include any type of structures, from a single absorbent layer to more complex multi layer structures. Certain absorbent articles include a fluid pervious topsheet, a backsheet, which may be fluid impervious and/or may be water vapour and/or gas pervious, and an absorbent core comprised there between.

The term "use", as used herein, refers to the period of time that starts when the absorbent article is actually put in contact with the anatomy of a wearer.

By "body fluid" it is meant herein any fluid produced by human body including, but not limited to, perspiration, urine, menstrual fluids, vaginal secretions and the like.

The term "aglycone derivable from and iridoid glycoside" indicates an aglycone (i.e. the non-sugar component of a glycoside molecule that results from hydrolysis of the molecule) which can be obtained from the hydrolysis of an iridoid glycoside, and includes aglycones obtained from other synthetic routes or directly extracted from natural sources.

An iridoid glycoside is a glycoside in which the aglycone is a compound belonging to the class of "iridoids", i.e. a monoterpene found in a variety of plants and animals. Iridoids usually comprise a cyclopentane ring fused to a six members oxygen heterocycle.

Aglycones derivable from an iridoid glycoside have the following general formula: 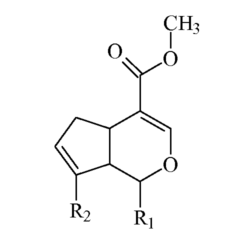 wherein R₁ is selected from a benzoyloxyl group, a hydroxyl group, an acetoxyl group and an ethoxyethoxyl group, and R₂ is selected from a benzoyloxymethyl group, a methoxymethyl group, a tert-butyldimethylsilyloxymethyl group, a carbonyl group or a hydroxymethyl group.

An exemplary aglycone for use in the present invention is genipin, an aglycone derivable from an iridoid glycoside according to the previous general formula, wherein R₁ is an hydroxyl group and R₂ an hydroxymethyl group:

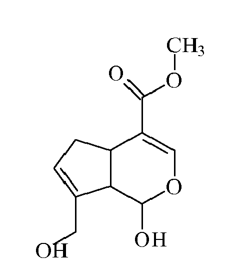

Genipin is an hydrolytic product of geniposide, a glycoside found in the fruits of Gardenia Jasminoides Ellis.

The present invention relates to an absorbent article comprising an aglycone derivable from an iridoid glycoside and/or one or more derivative thereof.

The absorbent article of the present invention may comprise an absorbent element intended to retain body fluids which may include natural or synthetic absorbent fibers or foams and/or one or more superabsorbent polymers. In some embodiments the aglycone derivable from an iridoid glycoside and/or one or more derivative thereof may be comprised within the absorbent element. In these cases the aglycone derivable from an iridoid glycoside and/or one or more derivative thereof may be incorporated in any manner available to the skilled man such as finely dispersed within the absorbent element and/or partially or totally absorbed within the absorbent fibers or the superabsorbent polymers of the absorbent element, or coated or printed on or within the absorbent element.

In some embodiments an absorbent article according to the present invention may comprise a fluid pervious topsheet, a fluid impervious backsheet and an absorbent element comprised between topsheet and backsheet. In these embodiments the aglycone derivable from an iridoid glycoside and/or one or more derivative thereof may be comprised in any or in any or all of topsheet, backsheet and absorbent element or may be comprised within additional elements interposed between them in any way which allow the contact of the aglycone derivable from an iridoid glycoside and/or one or more derivative thereof with bodily fluid upon use of the article.

The amount of aglycone derivable from an iridoid glycoside and/or one or more derivative thereof which is usually present in the articles according to the present invention is from 1 to 2500 mg per each absorbent article, or from 2 to 1000 mg per each absorbent article, or from 5 to 500 mg per each absorbent article, or from 10 to 250 mg per each absorbent article.

In some embodiments the article according to the present invention can be a feminine hygiene article like a sanitary napkin, an interlabial pad, a vaginal tampon or a pantyliner.

The aglycone derivable from an iridoid glycoside and/or one or more derivative thereof can be introduced within the absorbent article in any form, including in dry powder form, as a suspension in a liquid or as a solution.

An absorbent article according to the present invention has an improved capacity to retain body fluids, particularly proteinaceous or serous body fluids such as typically menses, blood, plasma, vaginal secretions, mucus or milk.

Without wishing to be bound by theory this is believed that the aglycone derivable from an iridoid glycoside and/or one or more derivative thereof acts as a cross linker for the proteins present in the body fluid so to cause a increase in viscosity and an increased retention of the crosslinked fluid within the absorbent article.

### Example

The viscosity of a sample of Artificial Menstrual Fluid is measured before and 3 hours after the addition of 5% w/w of Genipin (which corresponds to 250 mg per article, considering a mean load of 5 g of menstrual fluid per article). The viscosity is measured using a Brookfield DV III Viscometer equipped with a UL adapter, temperature is set at 23°C and data are elaborated using the software "Rheocalc v. 1.1.". The measurement is taken at 30 rpm (corresponding to a shear rate of 36.7 s⁻¹).
Results are as follows:

| | |
|---|---|
| AMF (ref) | 7.30 cP |
| AMF 5% genepin | 14.8 cP |

Te results obtained demonstrate that genipin is able to increase the viscosity of the menstrual fluid and therefore to aid its containment within the absorbent article

AMF is a fluid based on sheep's blood which is modified to ensure it closely resembles human menstrual fluid in viscosity, electrical conductivity, surface tension and appearance. AMF it is prepared as described in US Patent 6,417,424 assigned to The Procter & Gamble Company, from line 33 of column 17 to line 45 of column 18, to which reference is made.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article comprising an aglycone derivable from an iridoid glycoside and/or one or more derivative thereof having the following general formula: 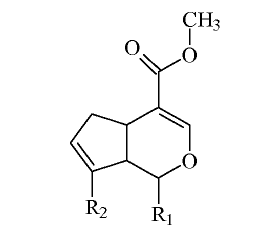 wherein R₁ is selected from a benzoyloxyl group, a hydroxyl group, an acetoxyl group and an ethoxyethoxyl group, and R₂ is selected from a benzoyloxymethyl group, a methoxymethyl group, a tert-butyldimethylsilyloxymethyl group, a carbonyl group or a hydroxymethyl group.

2. An absorbent article according to claim 1 wherein R₁ is an hydroxyl group and R₂ an hydroxymethyl group.

3. An article according to any preceding claim wherein the article is selected from absorbent articles for feminine hygiene.

4. An article according to claim 3 selected from a sanitary napkin, a pantyliner, a vaginal tampon or an interlabial pad.

5. An article according to any preceding claim comprising the aglycone derivable from an iridoid glycoside and/or one or more derivative thereof at a total level of from 1 to 2500 mg per each absorbent article, or from 2 to 1000 mg per each absorbent article, or from 5 to 500 mg per each absorbent article, or from 10 to 250 mg per each absorbent article.

6. The use of an aglycone derivable from an iridoid glycoside and/or one or more derivative thereof, having the following general formula: 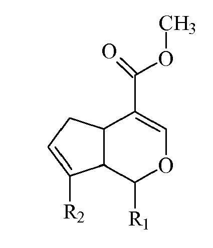 wherein R₁ is selected from a benzoyloxyl group, a hydroxyl group, an acetoxyl group and an ethoxyethoxyl group, and R₂ is selected from a benzoyloxymethyl group, a methoxymethyl group, a tert-butyldimethylsilyloxymethyl group, a carbonyl group or a hydroxymethyl group, in feminine hygiene absorbent articles to reduce leakage of menstrual fluids.
